# EUROPEAN PATENT APPLICATION

(11) **EP 3 578 094 A1**
(43) Date of publication of application: **11.12.2019**
(21) Application number: 17792945.2
(22) Date of filing: 01.03.2017
(51) Int. Cl.: A61B 5/00

(54) **METHOD FOR DETERMINING A PERSON'S CORONARY STATUS AND USE THEREOF TO MONITOR AND PREVENT THE RISK OF CARDIOVASCULAR DISEASES**

(30) Priority: 05.05.2016 RU 2016117464
(71) Applicant: Gabinskii, Ian Lvovich, Sverdlovskaya Oblast 624030 (RU)
(72) Inventor: GABINSKII Ian Lvovich, Beloyarskij eajon 624030 (RU); FREIDLIN, Marina Samuilovna, Ekaterinburg city 620026 (RU); YAKOVLEV, Yury Rengoldovich, Ekaterinburg city 620017 (RU); YAKOVLEVA, Svetlana Viktorovna, Ekaterinburg city 620017 (RU); KORKINA, Anna Yuryevna, Ekaterinburg city 620026 (RU)
(74) Representative: Jirkalova, Hana
(86) International application number: PCT/RU2017/050008
(87) International publication number: WO 2017/192066

(57) **Abstract**

The invention relates to medicine, to therapy, cardiology, in particular to the planning of prevention and treatment of patients at risk or already suffering from cardiovascular diseases (CVD).

The method of determining the coronary status of a person is carried out by determining the coronary code, presented in the form of a four-digit numeric entry, which is determined according to the visual assessment of the patient's coronary arteries using computed tomography, coronary angiography data, cardiovascular risk summary on the SCORE scale and data on cardiovascular risk factors for the additional EFIR coronary risk scale, data on the presence of percutaneous coronary intervention and / or coronary artery bypass surgery. Analysis of the data are mathematical methods using software. The final coronary code of a person is presented as a four-digit numeric entry. The determination of the coronary code of a person to one of the coronary groups GABICA-X is carried out by conducting a comparative analysis of four-digit numeric entries of coronary codes. The coronary status of a person is determined on the basis of the person's belonging to a particular coronary group. The totality of the data obtained is a human coronary passport. The application of the above method is carried out by arranging a visit, the primary determination of the coronary status by the method described above, and the implementation of appropriate therapeutic and preventive measures. The coronary status of a person is determined on the basis of the person's belonging to a particular coronary group. The totality of the data obtained is a human coronary passport. The application of the above method is carried out by arranging a visit, the primary determination of the coronary status by the method described above, and the implementation of appropriate therapeutic and preventive measures. Information about the coronary status and prescribed treatment-and-prophylactic measures are recorded on electronic media, for example, on a flash card, said electronic media is a means of patient nosological identification and a key to access the global information network, preferably the Internet. Through the specified key, an automated doctor-patient interaction is performed.

Technical result: creation of a universal automated system for monitoring and preventing the development of CVD.

## Description

### FIELD OF THE INVENTION

The invention relates to medicine, to therapy, cardiology, in particular, relates to the planning of prevention and treatment of patients at risk or already suffering from diseases such as cardiovascular diseases.

### BACKGROUND

Cardiovascular diseases lead the world by causes of death and disability, especially in view of the gradual trend towards aging of the planet's population. At the same time, there is a tendency to increase the number of people suffering from this pathology. According to the World Health Organization, 17 million people die each year from myocardial infarction and other diseases of the cardiovascular system.

It is well known that with age the risk of cardiovascular diseases increases, but in the modern world very young people are also often at risk. The lifestyle of most modern people leaves much to be desired: low physical activity, unhealthy diet, stressful situations, bad habits. As a result, diseases of the cardiovascular system are rapidly becoming "younger". Overweight, smoking, unhealthy diet, metabolic disorders, physical and psycho-emotional overload are the main causes of heart disease.

Unlike other diseases, the development of cardiovascular diseases can be prevented through simple on many occasions measures to change lifestyles. Nowadays, it is generally accepted that all people should strive for a healthy lifestyle in order to prevent cardiovascular diseases, but there are groups of people who have a higher risk of developing cardiovascular diseases, such as, for example, heart attack or stroke. Thus, it is desirable to effectively identify individuals who are vulnerable to cardiovascular diseases, and assign them to the prevention or treatment program aimed at including lifestyle changes, in order to improve human health.

The main modern problem is the presence of many cases of asymptomatic development of coronary heart disease, when the first manifestation of the disease becomes acute myocardial infarction or sudden coronary death. It is important to note that this group of people who are generally considered healthy, as they do not show any symptoms, should be attributed to an increased risk group. A way to monitor their cardiovascular health should be easy, time-consuming and inexpensive. In addition, since they feel healthy, an important task is the motivation to undergo diagnosis and treatment or lifestyle changes. The results of the state of coronary health should be presented in a way that allows people to understand the need to follow the advice of the doctor.

In order to develop a monitoring system for the patient's cardiovascular health, it is first necessary to determine the risk of the possibility of developing cardiovascular diseases. The patent and scientific literature describes the method of determining s risk of cardiovascular disease based on several parameters.

One of the existing methods for determining risks is Framingham scale, which is designed to calculate the risk of coronary death and total score of fatal and nonfatal coronary events in the next 10 years in men with cholesterol HDL 1,3-1,53 mmol / 1 and unavailable medications that lower blood pressure (according to the NCEP ATP from 3 2002) [Wilson PW, D'Agostino RB, Levy D., et al. Prediction of coronary heart disease using risk factor categories // Circulation. - 1998. - Nº12. - Vol. 97. - P. 1837-1847; Brindle P., Emberson J., Lampe F. et al. Predictive accuracy of the framingham coronary risk score in British men: prospective cohort study // BMJ. - 2003. - Vol. 325. - p. 1267-1270.]. This scale is not applicable to people with other levels of HDL cholesterol, and in assessing the risk of taking medications that lower blood pressure. In addition, the study was performed for the American population, and when used in other regions, the real absolute risk is overestimated [Brindle P., Fahey T.Primary prevention of coronary heart disease // BMJ. - Vol. 325. - p. 56-57].

The next major known method for identifying risks of developing cardiovascular diseases is the SCORE individual cardiovascular risk assessment scale (Systematic Coronary Risk Evaluation), which takes into account gender, age, smoking and education status, and total blood cholesterol concentration [Conroy RM, Pyorala K., Fitzgerald A.R., et al. SCORE project group. Risk of fatal cardiovascular disease in Europe: the SCORE project // Eur. Heart J. - 2003. - Vol. 24 (11). - P. 987-1003]. Developers of the SCORE scale indicated that the assessment of the total risk on this scale should be adapted for individual European populations depending on national conditions, resources and priorities. There is a variant of the scale adapted to the Russian population [Shalnova S.A., Oganov R.G., Deev A.D. Cardiovascular risk assessment and management for the Russian population // Cardiovascular therapy and prevention. - 2004. - Nº4. - P. 4-11], a distinctive feature of which is the inclusion of a discriminator specific for the Russian population in the number of predictive factors - the level of education, as well as a number of other factors (relative body weight, pulse rate, high density lipoprotein cholesterol level). Russia national scale, as well as European, predicts fatal events, and high-risk threshold is defined as a greater than 5% in a decade. Scale SCORE, ka to European and Russian, has limitations in application, since it can only be applied to individuals without clinical evidence of atherosclerotic disease that substantially limits scale use in actual practice. Patients with an established diagnosis of a cardiovascular disease, with diabetes mellitus and / or total cholesterol levels above 8.0 mmol / 1 or blood pressure greater than 180/110 mm Hg. are classified as high risk, and to them the calculation of the total risk by SCORE cannot be applied. Whereas in some patients with clinical manifestations of atherosclerosis, the disease has been stable for many years and does not end with any serious complications.

A disadvantage of the known methods for determining risk is that the prediction of individual risk does not ensure the absolute accuracy of the result. The accuracy of determining the risk of developing cardiovascular complications by these methods does not exceed 50% [Chobanian AV, Bakris GL, Black HR et al. Joint National Committee on Prevention, Detection, Evaluation, and Treatment of High Blood Pressure, National Heart, Lung, and Blood Institute, National High Blood Pressure Education Program Coordinating Committee: Seventh report of the Joint National Committee on Prevention, Detection, Evaluation, and Treatment of High Blood Pressure.// Hypertension. - 2003. - Vol. 42. - P. 1206-1252].

An assessment of the risk of developing cardiovascular diseases by known methods is calculated by a doctor using a program or table. The calculation of risks is used to determine the risk category for the development of a cardiovascular disease in humans: low, medium or high. Based on the risk category, determined by one of the known methods, treatment in accordance with world medical recommendations can be recommended. The generation of these recommendations for existing risk groups is disclosed in US Patent No. 4,464,122. This system of generating a summary in the form of comments and recommendations regarding the patient's health is intended to attract attention not only to the physical condition of the subject, but also aims to improve health, using the proposed changes in lifestyle.

Closest to the claimed solution is US patent No. 7,647,234, revealing a system that diagnoses and prescribes the treatment of cardiovascular diseases. Data is entered from the patient's card and with the use of an automated system, measures for prevention and treatment are recommended. This decision does not take into account patients who clearly cannot be attributed to the risk of developing cardiovascular diseases.

### SUMMARY OF THE INVENTION

The task of the invention is the creation of a universal automated system for monitoring and preventing the risk of developing cardiovascular diseases, suitable for detecting the risk of fatal cardiovascular complications in the early stages of a wide group of people, in particular those who have not previously been assigned to risk groups.

The technical result consists in increasing and effectiveness of a universal automated system for monitoring and preventing the development of cardiovascular diseases of a wide group of people based on a method for detecting the risk of developing cardiovascular diseases.

In general, the terms used in the description should be interpreted as known to those skilled in the art. Some terms are defined below in order to provide additional clarity. In the event of a conflict between a known value and the submitted definition, the submitted definition should be used.

The term chronic renal failure is understood to be chronic, renal failure.

The term CT is understood as multispiral computed tomography - angiography of the coronary arteries.

The term CAG refers to the coronary angiography of the coronary arteries.

The term PCI is percutaneous, coronary intervention.

The term CABG refers to coronary artery bypass surgery.

The term blood pressure refers to blood pressure.

The term OHS means the concentration of total cholesterol.

The term patients with diabetes mellitus type 1 and type 2 refers to patients with diabetes mellitus (DM) of the first and second types.

The term " coronary artery disease" ischemic heart disease.

The term cholesterol HDL refers to cholesterol with lipoproteins. high density.

The term CVD refers to cardiovascular diseases.

Under the term BMI is understood as body mass index.

The term "coronary code" refers to the classification of patients, carried out on the basis of a combination of visualization of the patient's coronary arteries with a total cardiovascular risk, determined by the proposed method, and is presented as a four-digit numeric entry.

The term "coronary group" means the classification of patient groups, determined on the basis of the patient's coronary code. Specified coronary groups assigned to name GABICA (Groups of Arterial Blockage Interpreted as Coronary Aggravation). The classification of coronary groups is recorded as GABICA - X, where X has values from 1 to 5. Groups of patients with visual manifestations of arterial disorders that lead to coronary complications in the future are included in the GABICA coronary group. When combining patients into a specific coronary group GABICA- X, the risk scale of fatal coronary complications EFIR (Extra Factors Increasing Risk) and the presence or absence of PCI and / or CABG intervention. In relation to the proposed method of forming the GABICA-X coronary groups, the corresponding P1-P10 coronary treatment programs have been developed for each group, taking into account therapeutic and preventive measures that significantly reduce the risk of future fatal complications in the next 2-3 years.

The term "coronary status" refers to the classification of patient groups, determined on the basis of the coronary patient code and belonging to the coronary group.

The term "coronary passport" means the proposed medical and technological principle of increasing the effectiveness of therapeutic and preventive measures to preserve the patient's coronary health and improve the quality of his life. The proposed method for a comprehensive assessment of the coronary status of a person is performed on the basis of modern non-invasive and minimally invasive methods for diagnosing coronary vessels and determining the total cardiovascular risk.

The invention includes a method of assessing the coronary status of a person and its use in the system of prevention and monitoring the risk of developing cardiovascular diseases.

The method for determining the coronary status of a person includes:
- determination of the coronary code, presented in the form of a four-digit numeric entry, determined by obtaining data from the patient's visual assessment of the patient's coronary arteries using computed tomography (CT), coronary angiography (CAG) data, cardiovascular risk summary data and factors risk of cardiovascular disease on an additional scale of coronary risk EFIR, the presence of percutaneous data coronary interventions (PCI) and / or aortocoronary shunting (CABG),
- subsequent analysis of the data obtained by mathematical methods using software,
- obtaining the final coronary code of a person, presented in the form of a four - digit numeric entry,
- determination of the coronary code of a person to one of the coronary groups by conducting a comparative analysis of four- digit numeric entries of the coronary codes,
- the establishment of the coronary status of a person on the basis of the person's belonging to a specific coronary group.

Application coronary status in the system preventing and monitoring the risk of cardiovascular disease, which includes registration visits primary definition coronary way status described above and carrying out therapeutic measures, taking into account the identified coronary status, with subsequent m determined HAND at predetermined intervals changing the time I coronary status and therapeutic and prophylactic x Events th, suitable x newly diagnosed coronary status data about the detected coronary status and assigned to treatment and preventive measures recorded on the electronic medium, which is a means of nosology human identification and access key to the global information lo be, preferably in the network Internet, through which the physician-patient interaction is automated, with the latter obtaining the latest research results, therapeutic- diagnostic measures and programs to prevent and / or treat.

The inventive method provides a comprehensive assessment of the coronary status of a person, allows you to implement new aspects of doctor-patient interaction, provides for the formation of programs for monitoring coronary status based on data on total cardiovascular risk on the SCORE scale and on an additional scale of risk factors for cardiovascular diseases EFIR, CT and CAG data, data on PCI and / or AKSH. The received source data is processed mathematically, for example, by cluster analysis methods, determining the coronary code in the form of a four- digit numerical record, by which the human coronary group is determined by comparing the obtained data with the data from one of the five specified GABICA-X coronary groups. By the coronary group of man determine its coronary status and choose the corresponding coronary treatment program, and the result of data processing is recorded on electronic media, which is the key to access global specialized information resources and the doctor-patient interaction tool.

In the study of scientific, technical and patent information, was not identified a set of essential features, identical to the stated in the present invention.

The following embodiment of the present invention will be described by way of example only with reference to the accompanying tables:
Table 1 - The structure of the coronary code and the interpretation of the values of bits in the numerical record of the coronary code.
Table 2 - Scale Description SCORE and EFIR scale description.
Table 3 - Coronary statuses and principles of classification of coronary groups.
Table 4- Group of indices for the formation of the first coronary group GABICA -1.
Table 5- Group of indices for the formation of the second coronary group GABICA -2.
Table 6- Group of indices for the formation of the third coronary group GABICA -3.
Table 7- Group of indices for the formation of the fourth coronary group GABICA -4.
Table 8- Group of indices for the formation of the fifth coronary group GABICA -5.
Table 9- Group of indices that are classified as a diagnostic error.
Table 10 - Description of coronary groups (GABICA-X).
Table 11 - Coronary treatment programs.

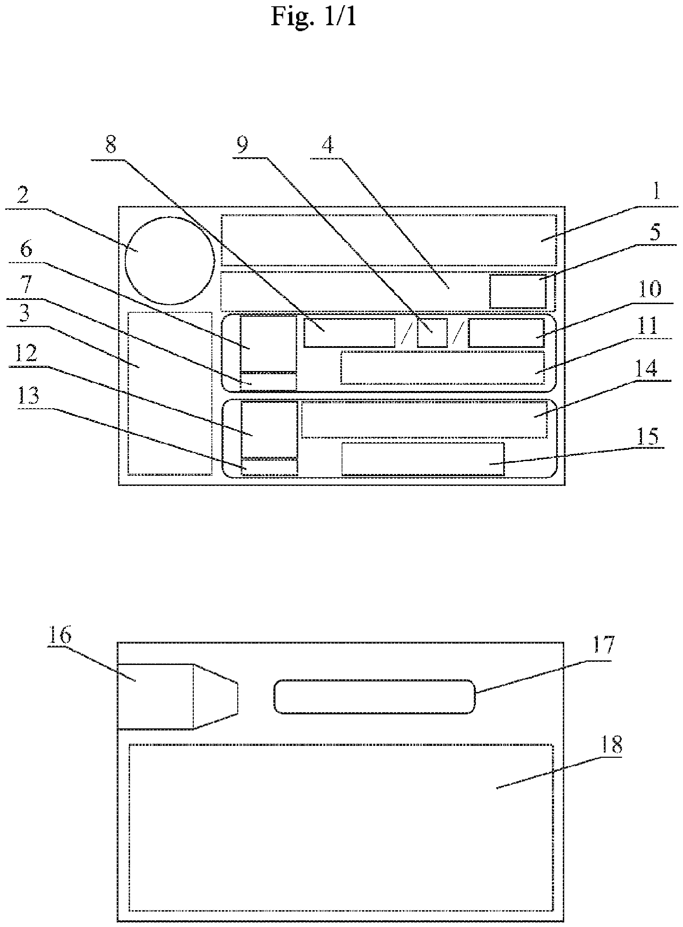

The invention is illustrated in FIG. 1, which schematically shows the appearance of a tool for identifying a human coronary passport in the form of an electronic plastic flash card.

The following detailed description of the invention is presented for its clearer illustration. However, it will be understood by those skilled in the art that the inventive concept of the present invention is not limited to these specific examples.

In one of the embodiments of the invention, the human coronary passport is made in the form of an electronic plastic flash card, schematically represented in FIG. 1, which contains: field 1 for recording contact information of the medical clinic, including the phone number and the address of the site of the medical clinic; field 2, intended for a holographic sticker (hologram) confirming the authenticity of the document; field 3 for recording instructions for the patient to use his coronary passport. The instructions for use preferably contain a minimum set of rules, such as the following:
- Without a hologram, a coronary passport is not valid.
- Medical relevance 2 years.
- To read the diagnosis, you need to scan the code.
- To enter the site on your personal account, you need to scan the code.
Zone 4 for inputting electronic media title, e.g. coronary SHEET (CORONARY PASSPORT) and field 5 to enter the serial number e plastic flash card, e.g. Nº 1266. Field 6 to enter information about the patient's diagnosis, provided, for example in QR code format; field 7 for entering the word "diagnosis"; a field 8 for entering a record of the patient's coronary group, including, inter alia, his management tactics, for example, GABICA -1; field 9 to enter information about the treatment and prevention program; field 10 for entering information about the coronary status of the patient; field 11 for entering information about the date of examination and the date of the next patient admission; field 12 to enter a link to the website of the medical clinic to enter personal account presented, for example, in a QR code format; field 13 to enter the word "site"; field 14 for entering patient information, such as name, gender, date of birth; a field 15 for entering patient account data to enter a clinic site, for example, a username and password; a memory card 16, which contains, for example, an electronic patient record, with patient test results recorded on an electronic medium; field 17, intended for the patient's handwritten signature; mechanical slider 17 for extending from the case of the electronic flash card of the contact pad of the USB type connector ; field 18 for entering information regarding consent to the processing of personal data.

Coronary status includes information about the patient's coronary arteries, the total cardiovascular risk and the presence of PCI and / or CABG interventions, which are necessary to prevent, diagnose and monitor coronary health.

According to the proposed method for determining the coronary status, 5 (five) coronary groups are distinguished, which differ in the risk of developing CVD. Based on the identification of the coronary group of a person to one of the specified coronary groups, patients are monitored and treatment-and-prophylactic measures are taken that are carried out according to 10 coronary treatment programs, at least one of which corresponds to the coronary status of a particular patient. Record of the coronary status can be carried out, for example, as follows.

The coronary status is recorded, for example, in the form of a coronary code represented by a four - digit numeric entry, denoted by digits, where:
- the first digit of the discharge (from 0 to 4) indicates the result of visualization of the coronary arteries according to computed tomography (CT).
- the second digit of the discharge (from 0 to 4) denotes the result of visualization of the coronary arteries by the method of coronary angiography (CAG).
- the third digit of the discharge (from 0 to 3) denotes the result of the total determination of the absolute risk of fatal cardiovascular complications on the SE scale, which summarizes the risks on the SCORE and EFIR scales.
- the fourth digit of the discharge (from 0 to 3) reflects the presence of interventions of PCI and / or CABG in history.

Interpretation of the values of each digit of the numeric entry of the coronary code is presented in Table 1.

In particular, a four- digit numeric entry of the coronary code in the form "1021" means that the first digit contains the digits of "1" and indicates the generalized CT result of the study, selected from possible values (0/1/2/3/4); the second digit contains the digits of "0" and indicates the generalized result of the CAG study, selected from the possible values (0/1/2/3/4), the third digit contains the digit "2" and points to the groups at risk, the definition of the combined scale of SE, that assesses the total risk by SCORE scale and EFIR; fourth category comprises digits have " 1" and indicates the presence / absence of PCI interventions and / or a history of CABG, selected the possible values (0/1/2/3).

The coronary code forms the coronary status, which is determined through the coronary group.

The principles of classification of coronary groups are summarized in Table 3. Each value in the table is represented by a group of four indices, each index reflects the values of the corresponding two columns and two rows of the table, recorded, for example, in the form "0100", which corresponds to the following indicators -CT- "0 ", KAG-" 1 ", SE -" 0 ", the intervention of PCI or / and CABG -" 0 «.

In Table 3, the columns reflect the CAG data and the risk values determined by the combined SE scale, which summarizes the risks on the SCORE and EFIR scales.

The absolute risk of fatal cardiovascular complications is determined by the combined SE scale, which summarizes the risks on the SCORE and EFIR scales. The SCORE scale description and the EFIR scale description are shown below and in Table 2.

The SCORE scale is designed to calculate fatal cardiovascular complications. Fatal cardiovascular complications (events) include: death from myocardial infarction, other forms of coronary heart disease, stroke, including sudden death and death within 24 hours after the onset of symptoms, death from other non - coronary cardiovascular diseases except definitely non-atherosclerotic causes of death. The SCORE scale is not used in patients with proven cardiovascular diseases of atherosclerotic origin (coronary artery disease, cerebrovascular diseases, aortic aneurysm, atherosclerosis of peripheral arteries), diabetes mellitus type I and II with target organ damage, chronic kidney disease, in individuals with very high levels of individual risk factors, citizens over the age of 65 years (these groups of people have the highest degree of total 10-year cardiovascular risk) and citizens under 40 years of age, because risk takers (with the exception of very high levels of individual factors) they have a low absolute risk of fatal cardiovascular complications in the next 10 years of life. The SCORE scale was developed based on the following factors: age, gender, smoking, systolic blood pressure and total cholesterol level.

According to the classification of risks according to the SCORE scale, all patients are divided into 4 groups:
1- low risk less than 1%
2- average risk from > 1 to 5%
3- high risk from> 5% to 10%
4- very high risk> 10%

The SCORE scale is intended for patients between the ages of 40 and 60 years.

Automatically (without calculation on the SCORE scale) the category of very high risk recommendations include patients with cardiovascular diseases of atherosclerotic origin (coronary heart disease, cerebrovascular disease: ischemic stroke, cerebral hemorrhage, transient ischemic attack, aortic aneurysm, clinically atherosclerosis peripheral arteries); patients with type 1 and type 2 diabetes with target organ damage (microalbuminuria); patients with chronic renal failure and symptoms of renal failure from moderate to severe (glomerular filtration rate (GFR) <60 ml / min / 1.73 m2); patients with arterial hypertension of 3 (third) degree (blood pressure> 180/110 mm Hg); patients with familial hypercholestenmia ; aged> 65 years; patients with total cholesterol numbers> 8 mmol / l; with heart failure, including heart failure with intact ejection fraction from stage II according to Strazhesko- Vasilenko classification .

Not included in the coronary code, but counted in the 3rd digit in the combined SE scale is the additional EFIR scale, reflecting the cardiovascular risk, depending on the influencing risk factors not included in the SCORE scale. Using the SCORE scale is likely to underestimate the risk of cardiovascular diseases, due to the fact that important risk factors for atherosclerosis are not taken into account. Therefore, it is necessary to identify a cardiovascular risk depending on the factors of asymptomatic organ damage. Evaluating total cardiovascular risk is key to choosing a preventive strategy for individuals who typically have a combination of several risk factors. Risk factors mutually potentiate each other. Even with low levels of individual risk factors, the overall risk can be significant. This is especially important in young patients (≥ 30 to 40 years) who are not included in the SCORE scale, as well as for patients with a low risk on the SCORE scale.

All patients on the EFIR scale are divided into 2 groups - low risk of CVD and high risk of CVD:
1 - low-risk group, which includes patients who scored less than 5 points;
2 - high-risk group, which includes patients who score 5 or more points.

If the SCORE scale defines the risk as low (<1 %) or medium (≥1% <5%), then they additionally evaluate the indicators on the EFIR scale, and depending on the score, a risk category is assigned as a total risk indicator on two scales. The scale of risk categories for our proposed classification of EFIR includes estimates that can vary from 0 to 8 points.

Indicators that are evaluated in assessing cardiovascular risk depending on risk factors, asymptomatic organ damage:
- plasma glucose on an empty stomach - 5.6-6.9 mmol /l - 1 point;
- increase of C-reactive protein (> 5 mg /l) - 1 point;
- body mass index> 30 kg / m² - 0.5 points;
- abdominal obesity (waist volume > 100 cm) - 1 point;
- lipoproteins of high density (HDL) <1, 0 in men and <1.2 in women - 1 point;
- subclinic lesion of the carotid arteries (stenosis> 50%) - 0.5 points;
- hereditary history of early cardiovascular diseases in relatives of the first line at the age of <45 in men, <55 years in women - 1 point;
- hypodinamia - 0.5 points;
- the presence of left ventricular hypertrophy; 0.5 points;
- professions associated with increased psycho- emotional overloads (drivers, pilots, firefighters, machinists, reporters, etc.) - 1 point;
   - family position (single men) - 0.5 points;
      depression - 0.5 points.

Depression, for example, can be rated on the well-known Beck scale.

The lines in Table 3 reflect the risks associated with these CT and interventions of PCI or / and CABG. It was empirically found that in Table 3 there are groups of indices in larger type, which are technologically most likely in medical practice (146 units). Of these are made up of four main coronary groups. In particular, the first coronary group is recorded as GABICA -1, the second coronary group is recorded as GABICA -2, the third coronary group is recorded as GABICA -3, the fourth coronary group is recorded as GABICA -4.

The groups of indices constituting the first coronary group GABICA -1 are enclosed in rectangular frames and shown in Table 4. The groups of indices constituting the second coronary group GABICA -2 are enclosed in rectangular frames and shown in Table 5. The groups of indices constituting the third coronary group GABICA -3, enclosed in rectangular frames and shown in Table 6. The groups of indices constituting the fourth coronary group of GABICA -4 are enclosed in rectangular frames and shown in Table 7. The remaining groups of indices shown small print, unlikely and considered to be outside the range of values to be considered (254 pcs.). Such coronary statuses are adopted as atypical, each must be treated individually, and take account of what s smiling as the fifth group coronary GABICA -5 (group index enclosed in a rectangular frame and are shown in Table 8), or as a violation of the technology that could lead to diagnostic errors (a group of indexes enclosed in rectangular frames and shown in Table 9). The definition of the index group belonging to the fifth coronary group GABICA -5 is carried out on the basis of the mismatch of the risk index SE and CT data, as a result of which it is necessary to revise the SE risk index and prescribe an individual coronary treatment program P10 (Table 11).

The resulting coronary codes are used to form the coronary status of the patient. As was shown above, during the formation of the patient's coronary status, patients are grouped according to the degree of coronary risks into the GABICA -1 ÷ GABICA- 5 coronary groups, described in Table 10. Coronary groups of patients are based on combining the results of CT and CAG studies, factors the risk of fatal cardiovascular complications interpreted on the basis of visual manifestations of coronary arteries. Therapeutic and prophylactic measures (coronary treatment programs P1-P10 are presented in Table 11) are determined on the basis of the patient's belonging to a specific coronary group GABICA -1 ÷ GABICA -5. Formation of patients in coronary groups, taking into account all factors that may affect the development of the disease and its complications, significantly reducing the risk of fatal coronary complications as a result of underreporting of significant coronary factors.

An automated doctor-patient interaction system for determining coronary status and obtaining medical prescriptions and recommendations includes:
1. Making visits and booking reception times, using an Internet portal, an online registry, and a patient's personal account, through the use of global services, preferably the Internet to access the pre-appointment log;
2. Registration of services, namely financial and legal registration, appointment of the patient's reception time;
3. Inspection and questioning of the patient, on the basis of which the formation of groups of patients is made: general technological and special;
4. The study of visualization of the coronary arteries according to multispiral computed tomography - coronary artery angiography (CT), and Coro angiography (CAG) followed by the interaction of the CT and CAG units with the hardware-software complex containing the database in the form of a medical information system, for example, a medical information system "MEDOFIS" (MIS "MEDOFIS"), and a computer program for processing raw data;
5. Transfer of initial data to the database and automatic filling of the protocol and medical record of the patient;
6. Processing of the initial data using mathematical analysis methods using a computer program and determining the patient's coronary status based on the results of data processing;
7. The conclusion of the doctor on the coronary status, on the basis of which the coronary group of the patient is determined and from the set of coronary treatment programs P1-P10, the coronary treatment program is selected corresponding to the coronary group of the particular patient.
8. Formulation of medical appointments and recommendations for treatment and preventive measures that are recorded in an electronic medical record of the patient and contain a description of the type of coronary treatment program and the scope of its implementation (monitoring, prevention, treatment or rehabilitation).

It should be noted that the above options only illustrate and not limit the invention.

### INDUSTRIAL APPLICABILITY

The technical result is that the present invention is a universal automated system that allows you to identify the risk of developing cardiovascular diseases (CVD), plan treatment and recommend lifestyle changes for all people, in particular those who have not previously been assigned to risk groups to prevent and prevent CVD. The use of the invention significantly reduces the risk of future fatal coronary complications in patients.

**Table 1**

| Structure of the coronary code and the interpretation of the values of the bits in the numerical record of the coronary code | | |
|---|---|---|
| **Digits of numeric entry** | **Value** | **The rule of assignment to the category** |
| 1st digit | 0 | Unreliable or impossible to obtain reliable information about the state of the coronary arteries using CT-CAG (due to rhythm disturbance, due to severe calcification) |
| shows the generalized result of visualization of the coronary arteries according to MS CT-CAG | 1 | CT-CAG with no signs of atherosclerotic changes in the coronary arteries (primary prevention) |
| | 2 | Initial changes in the coronary arteries without significant stenoses (the left main artery is less than 30%, other CA is less than 50%) (conservative treatment, dynamic observation, if necessary, repeated CT-CAG after 6 months) |
| | 3 | On CT-CAG stenoses requiring invasive angiographic control (border stenosis - 50 - 69%, lesions of the trunk - 30-49%) (planned CAG) |
| | 4 | According to CT-CAG, significant stenosis and occlusion of the coronary arteries. Mouth lesions, PKA lesions, LCA lesions (emergency CAG) |
| 2nd digit | 0 | CAG is not carried out |
| shows the generalized result of coronary artery imaging according to CAG | 1 | No change in coronary arteries based on CAG results. |
| | 2 | On the CAG changes in the coronary arteries that do not require invasive intervention (stenting) |
| | 3 | According to the CAG, the patient requires percutaneous intervention (stenting, angioplasty) |
| | 4 | According to the CAG results, the patient requires surgical treatment (CABG) |
| 3rd digit | 0 | Low risk less than 1% |
| combined scale SE, which summarizes the risks | 1 | Average risk from> 1 to 5% |
| | 2 | High risk from> 5% to 10% |
| (SE = SCORE * scale + EFIR scale **) | 3 max | Very high risk> 10% |
| 4th digit | 0 | No interventions |
| the presence of interventions PCI or/ and CABG | 1 | Stents have been installed |
| | 2 | CABG was performed |
| | 3 | Stents have been established and held CABG |

**Table 2**

| **SCORE scale description and EFIR scale description** | | |
|---|---|---|
| **Scale Name** | **Value** | **Value description** |
| SCORE scale | 0 | Low risk less than 1% |
| | 1 | Average risk from> 1 to 5% |
| | 2 | High risk from> 5% to 10% |
| | 3 | Very high risk> 10% |
| EFIR scale | 0 | Low risk - patients scored <5 points |
| | 1 | High risk - patients who score ≥ 5 points to 9 points |

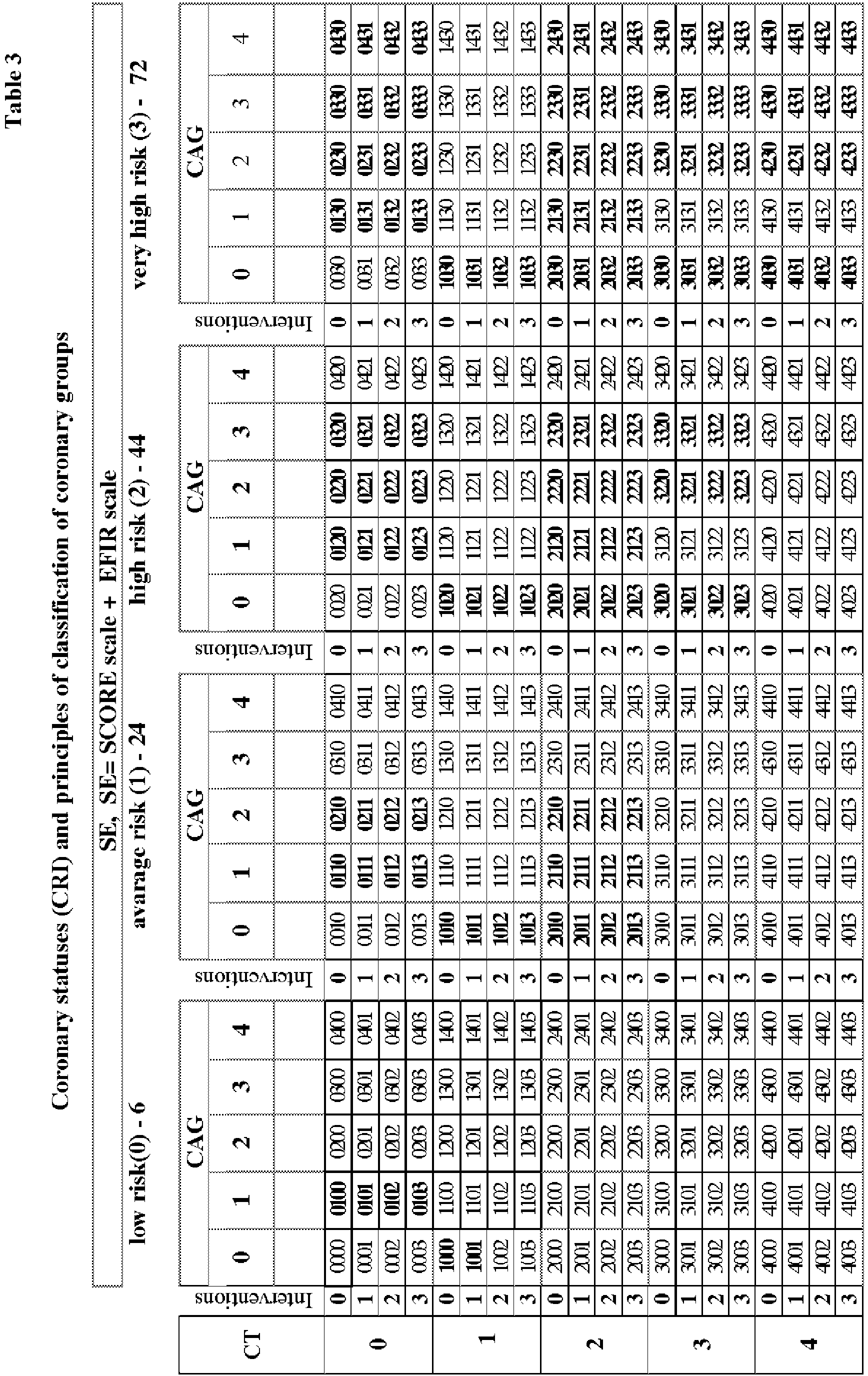

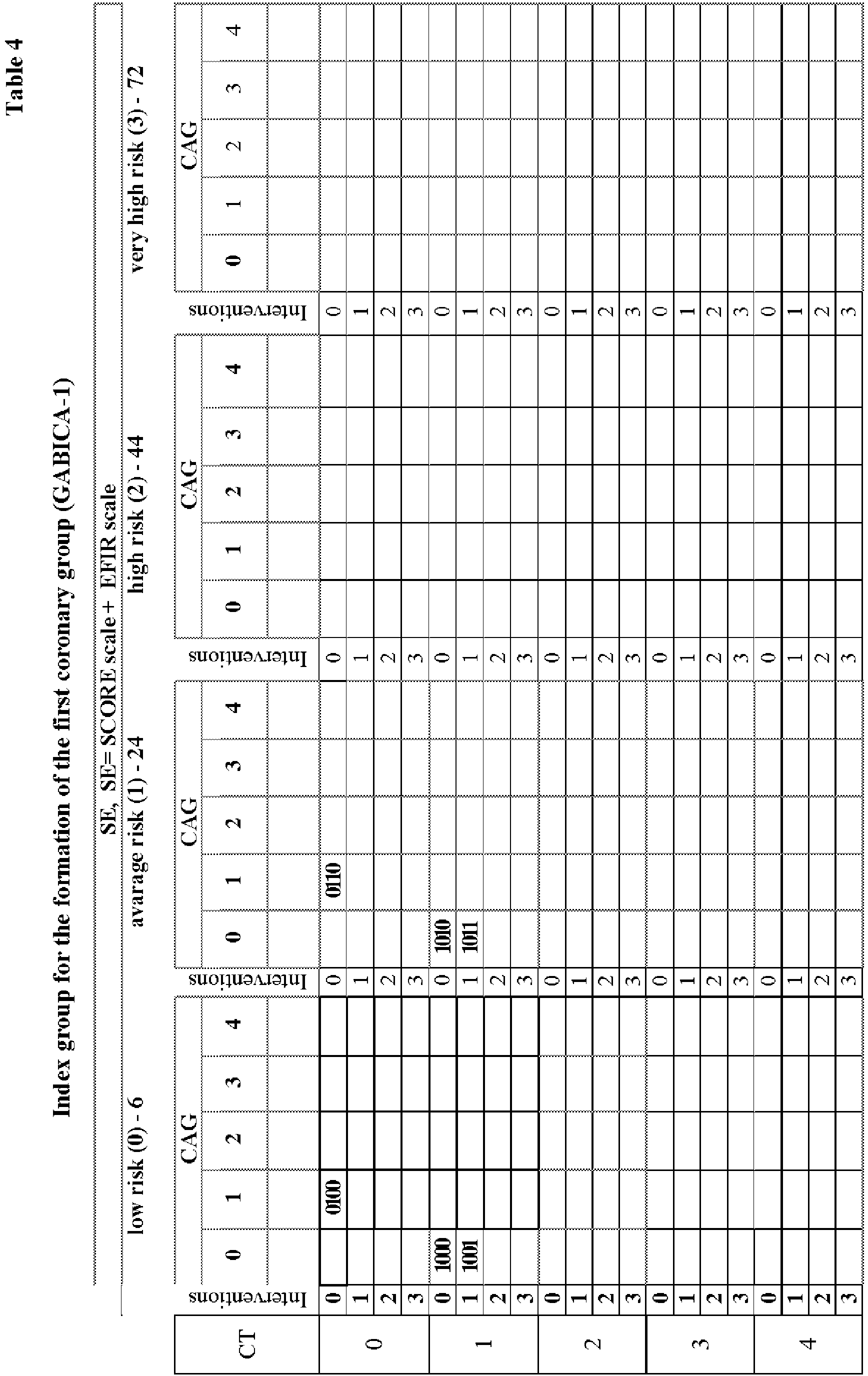

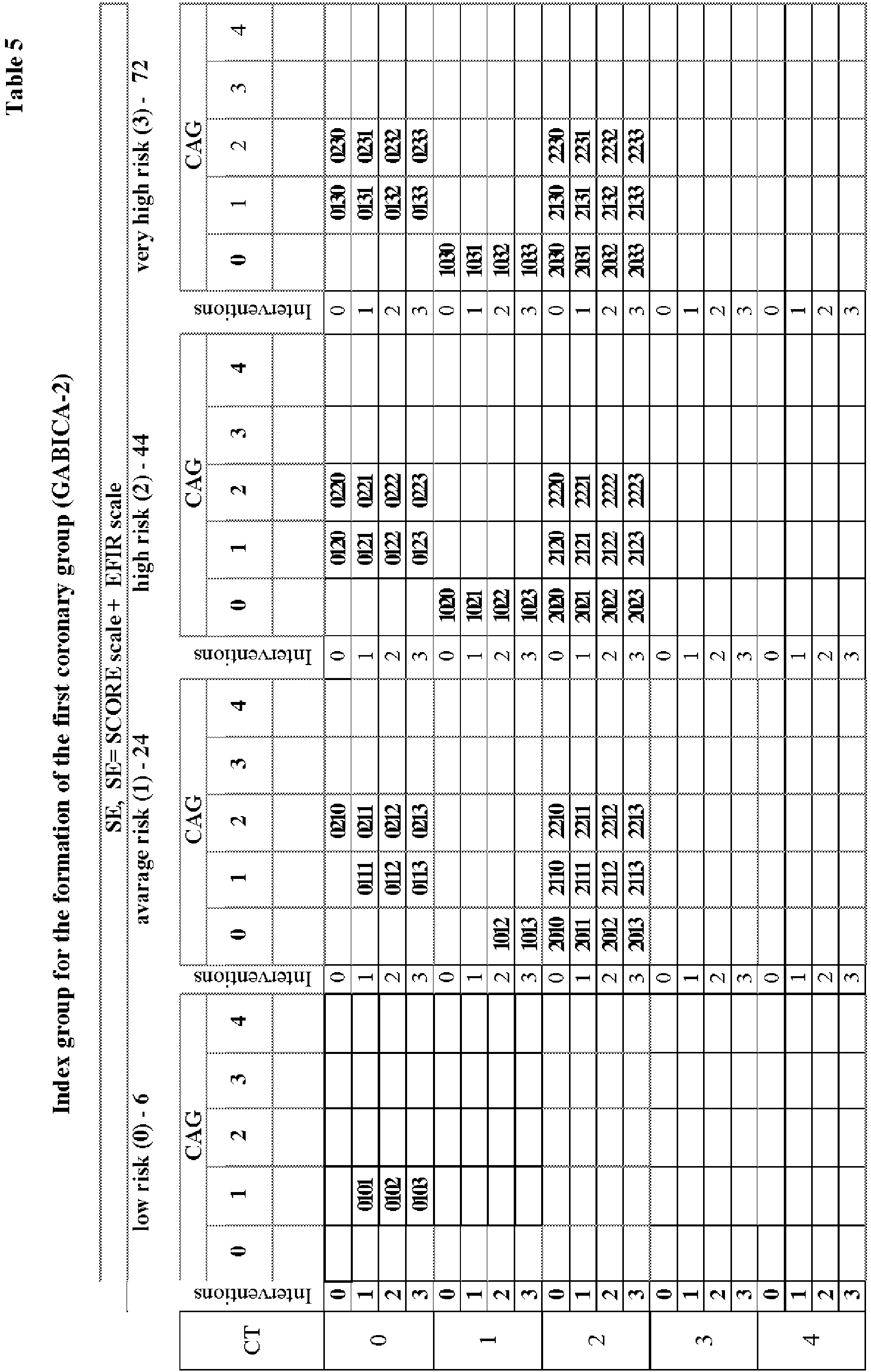

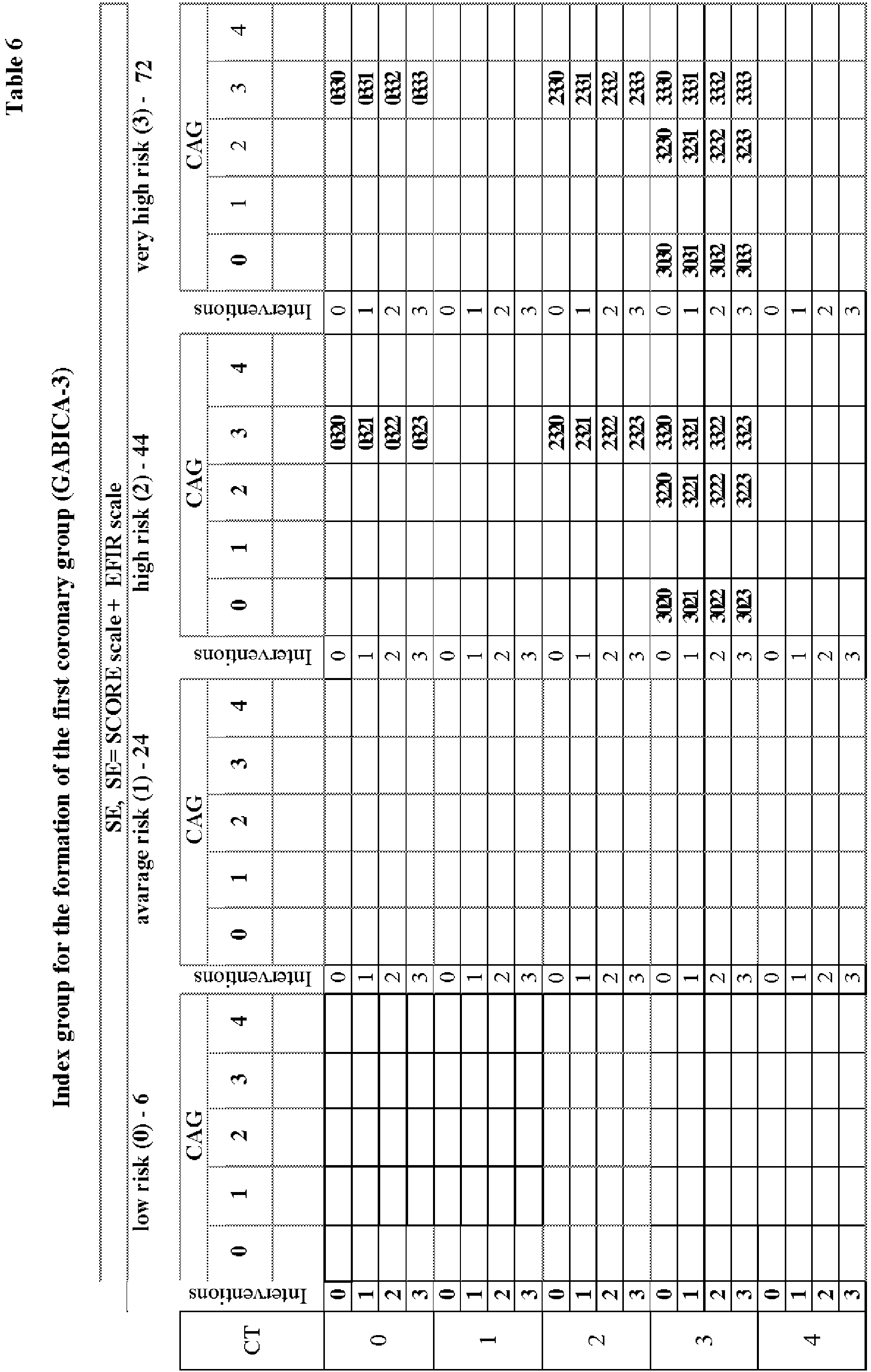

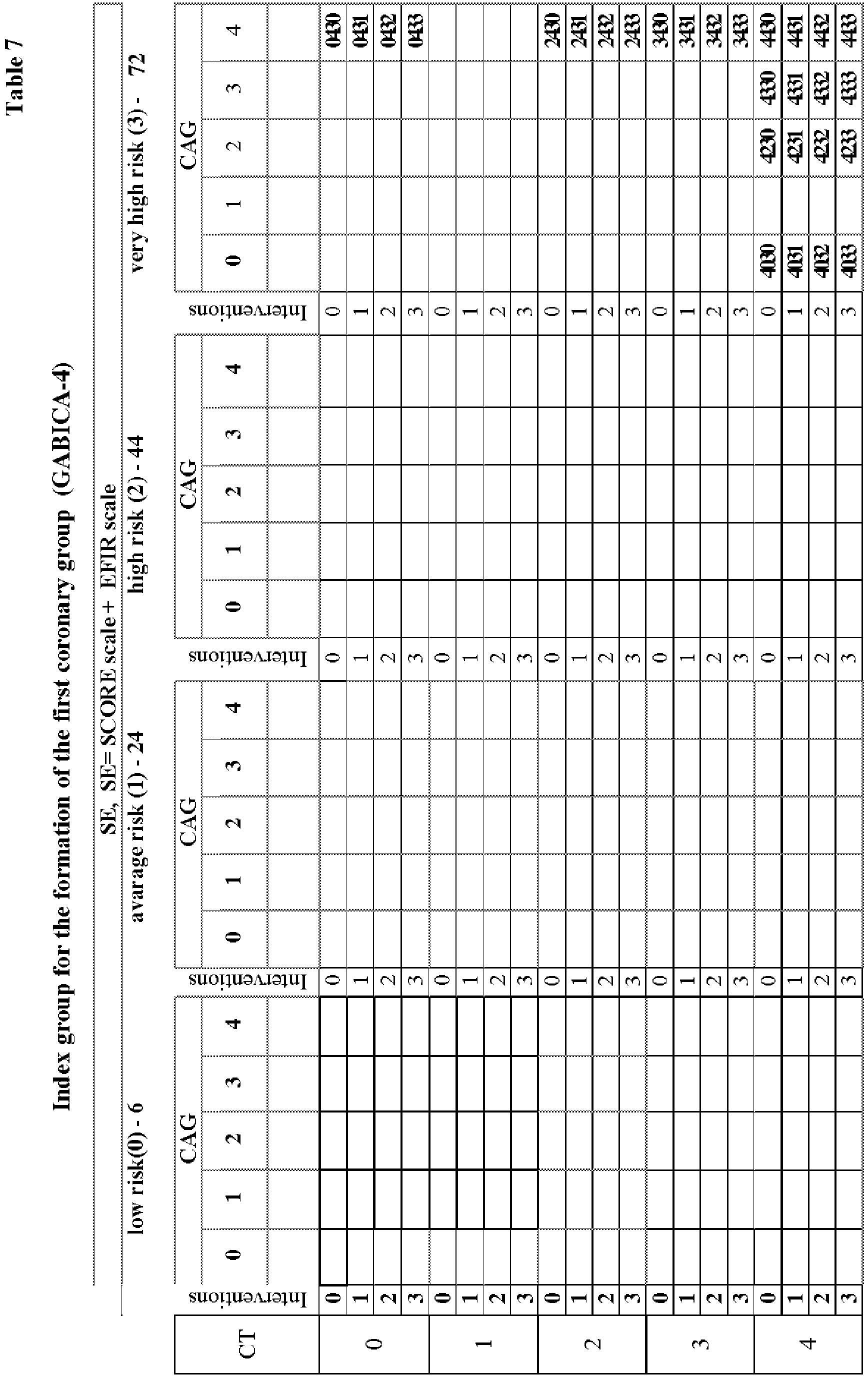

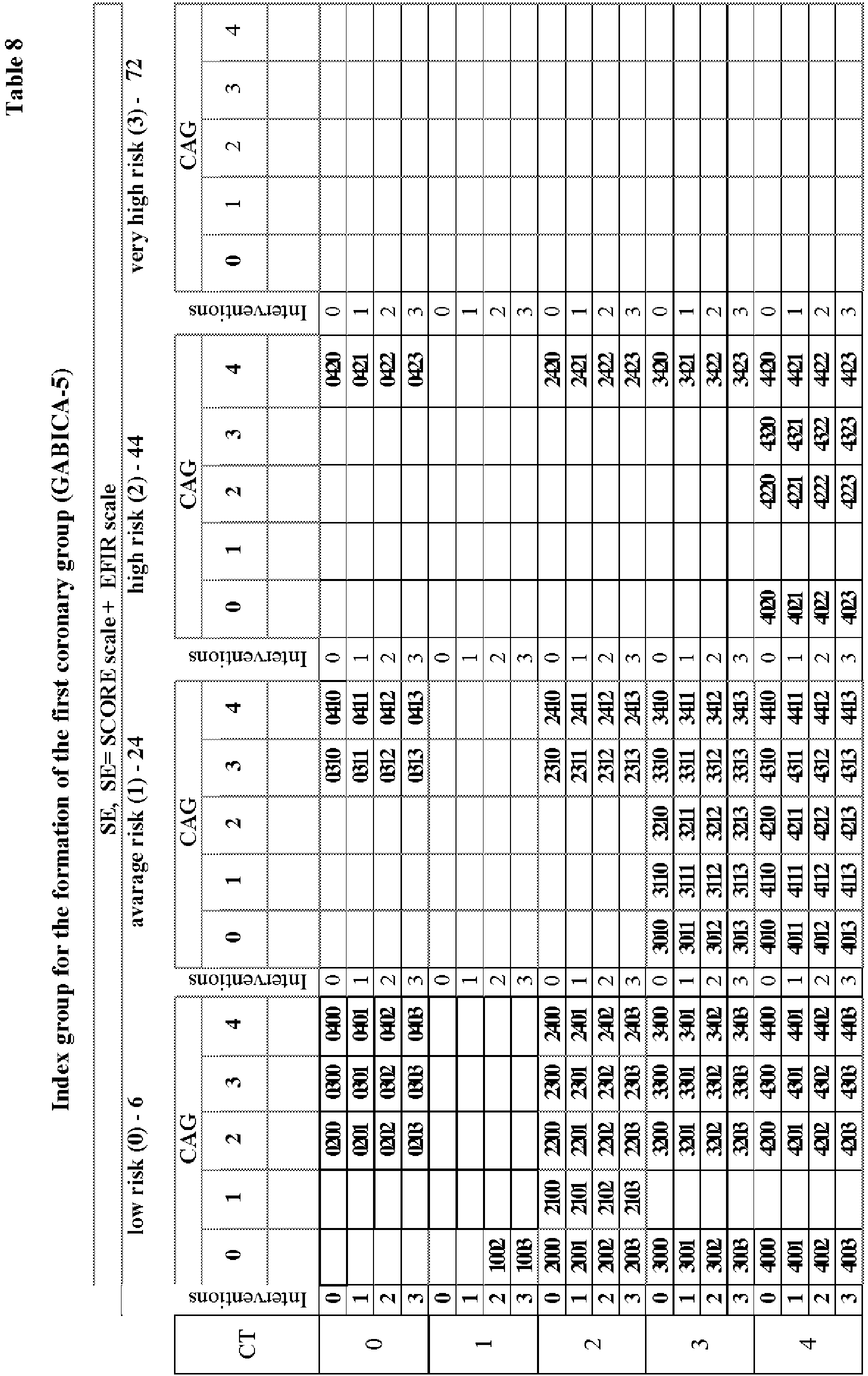

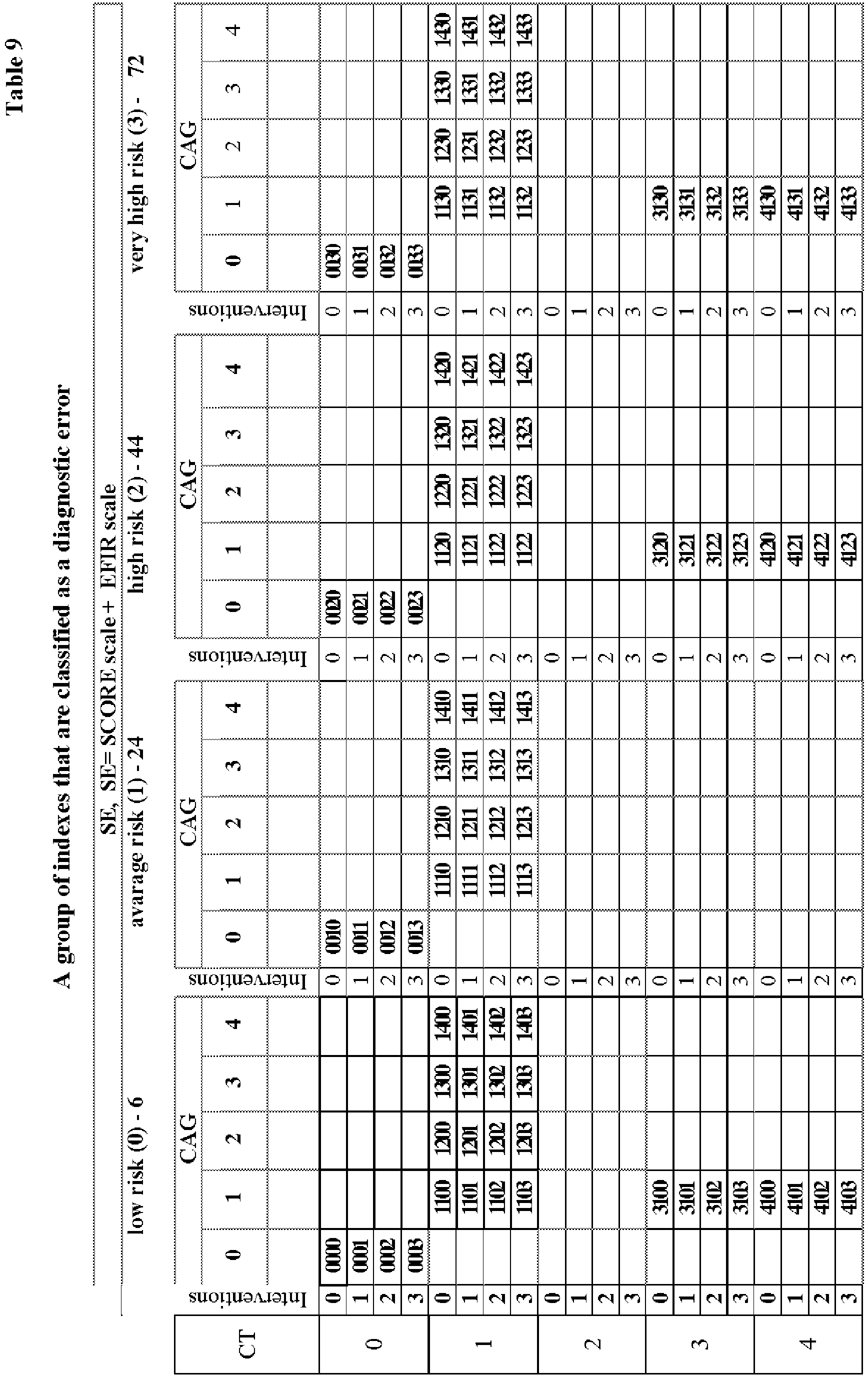

**Table 10**

| **Description of Coronary Groups** | | | | |
|---|---|---|---|---|
| **Coronary Groups** | | **SE risk group** | **The frequency of passage of CT-CAG** | **Coronary treatment program** |
| **No. of coronary group** | **Patient management tactics** | | | |
| GABICA-1 low risk | Preventive measures to maintain health indicators | 0 | once ever 5 ears | P1 |
| | | 1 | once ever 3 ears | P2 |
| | | | | |
| GABICA-2 avarage risk | Therapeutic and preventive measures with no need f or PCI or surgical interventions | 0 | once ever 2 ears | P3 |
| | | 1 | once a year | P4 |
| | | 2 | once a year | P5 |
| | | 3 | once a year | P6 |
| | | | | |
| GABICA-3 high risk | Therapeutic and preventive measures in preparation for invasive interventions | 3 | once a year | P7 |
| | | 4 | once every 6 months | P8 |
| | | | | |
| GABICA-4 very high risk | Therapeutic and preventive measures in preparation for surgical interventions | 4 | once every 6 months | P9 |
| | | | | |
| GABICA-5 atypical patients | Review the SE value, otherwise prescribe an individual examination schedule and therapeutic and preventive program | any | individually determined, but at least once every 5 years | P10 individual |

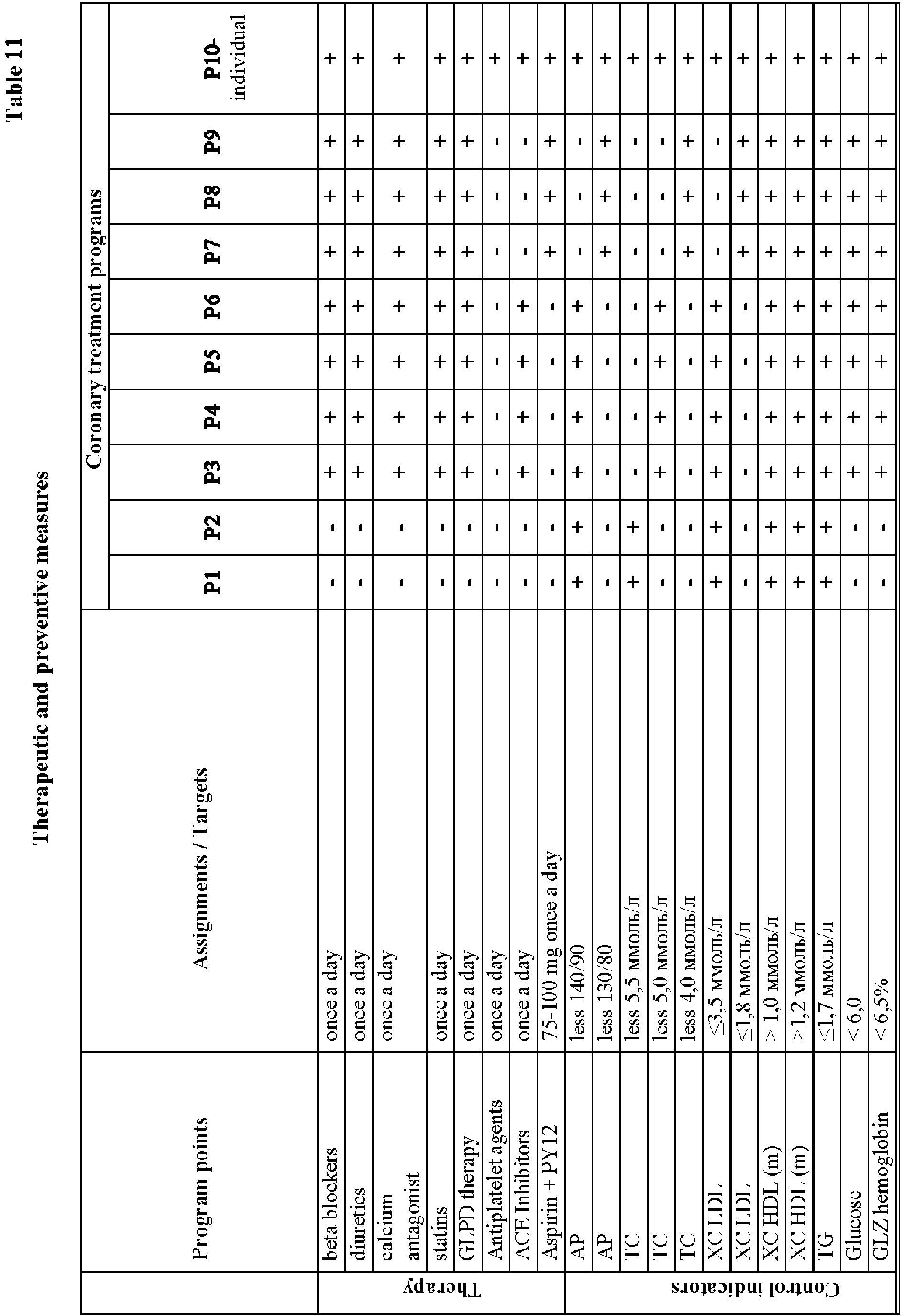

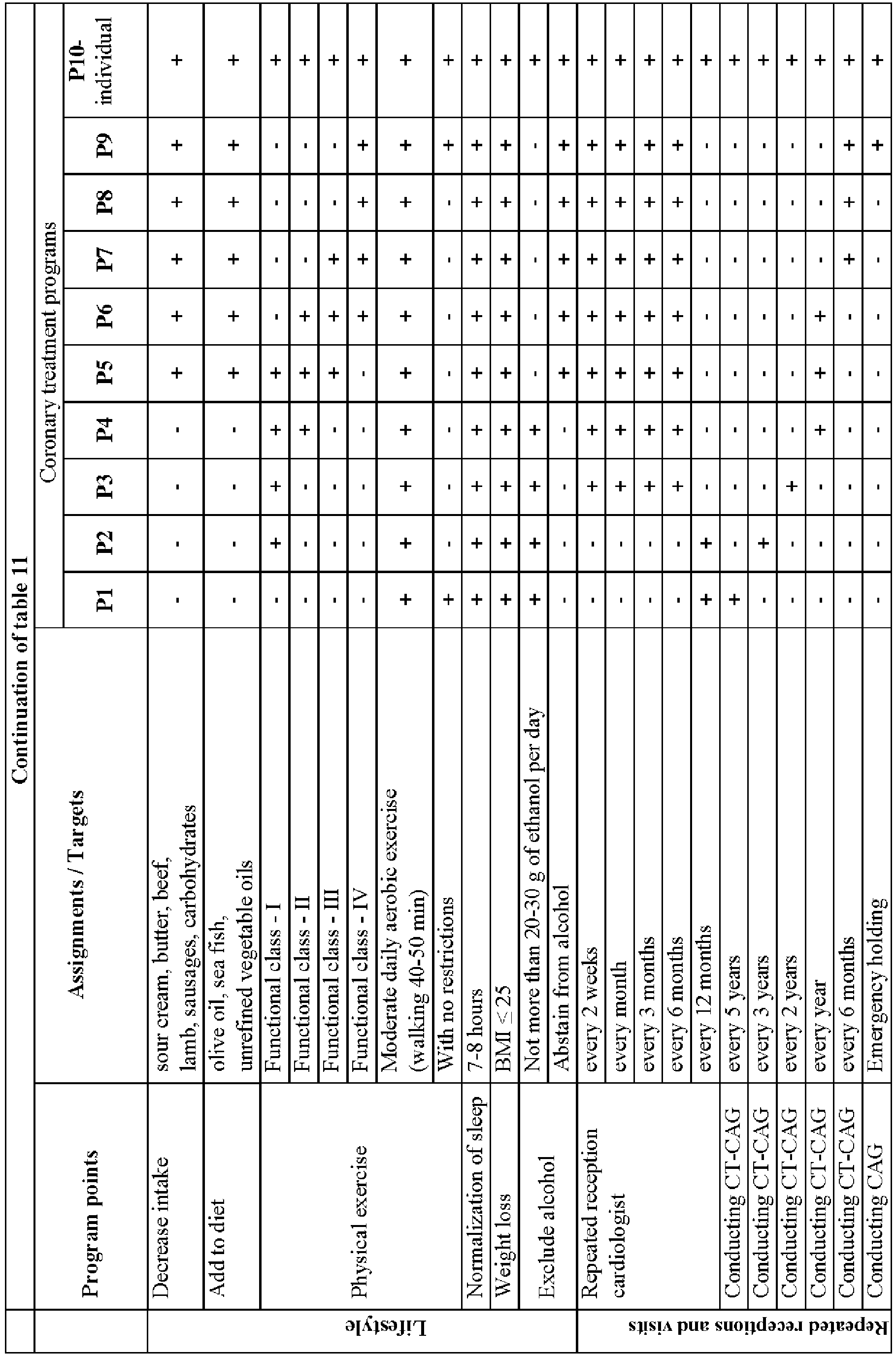

## Claims

1. The method of determining a person's coronary status is carried out by determining the coronary code, presented in the form of a four-digit numeric entry, determined by obtaining data of a visual assessment of the patient's coronary arteries using computed tomography, coronary angiography data, total SCORE risk data and risk factors for cardiovascular diseases on the additional scale of coronary risks of EFIR, and the EFIR scale contains:
1- low risk group, which includes patients who scored less than 5 points, and which has a value of 0;
2- high-risk group, which includes patients who score 5 or more points, and which has a value of 1,
moreover, the calculation of points is based on the evaluation of indicators:
- Fasting plasma glucose - 5.6 - 6.9 mmol/l- 1 point;
- increase of C-reactive protein> 5 mg/l - 1 point;
- body mass index> 30 kg / m - 0.5 points;
- abdominal obesity with waist volume> 100 cm - 1 point;
- high density lipoproteins <1.0 for men and <1.2 for women - 1 point;
- subclinical damage to the carotid arteries with stenosis> 50% - 0.5 points;
- hereditary history of early cardiovascular diseases in relatives of the first line at the age of <45 in men, <55 years in women - 1 point;
- hypodynamia - 0.5 points;
- the presence of left ventricular hypertrophy - 0.5 points;
- professions associated with elevated psycho-emotional overloads - 1 point;
- marital status - 0.5 points;
- depression, rated on the scale of Beck - 0.5 points,
data on the presence of percutaneous coronary intervention and / or coronary artery bypass surgery, the subsequent analysis of the data obtained by mathematical methods using software tools, obtaining the final coronary code of a person, presented in the form of a four-digit numeric entry, determining the coronary code of a person to one of the coronary groups by conducting a comparative analysis of four-digit numerical records of coronary codes, establishing the coronary status of a person based on the person's belonging to a specific coronary group.

2. The method of determining a person's coronary status according to claim 1, **characterized in that** the software for conducting research and collecting anamnesis is used in hardware-software complexes.

3. The use of coronary status for the prevention and monitoring of the risk of cardiovascular diseases, which includes registration of the visit, the primary determination of the coronary status by the method described in paragraph 1 and the implementation of therapeutic and preventive measures taking into account the identified coronary status, with a subsequent determination at specified intervals of changes in the coronary status and the appointment of therapeutic and preventive measures corresponding to the newly identified human coronary status, the data on the identified coronary status and the assigned therapeutic and preventive measures are recorded on electronic media, which is a means of nosological identification of a person and a key to access the global information network, preferably the Internet, through which the doctor-patient interacts automatically with the latest research results, therapeutic and diagnostic measures and prevention and / or treatment programs.

4. Application in the risk of coronary status monitoring system and the prevention of cardiovascular disease according to claim 3, **characterized in that** the doctor-patient interaction system includes the following operations:
**a.** scheduling an appointment and booking an appointment time, using an Internet portal, online registration, and the personal account of the patient, making use of global services for access to the appointments journal;
**b.** scheduling of services by the patient, specifically financial-legal registration, confirmation of appointment time;
**c.** patient examination by the doctor and filling out forms, on the basis of which the patient groups are formed: general technological and special;
**d.** entering by doctor of results of investigation of visualization of coronary arteries according to multispiral computed tomography - angiography of the coronary arteries and coronoangiography, followed by informational interaction of the CT unit and the coronary artery device with the medical information system with the transfer of the initial data to the database of the medical information system for filling the protocol;
**e.** automatic filling out of patient's medical chart;
**f.** determination of the coronary group and the final conclusion on the coronary status with the appointment of therapeutic and preventive measures.
